Europäisches Patentamt

European Patent Office

Office européen des brevets

(11) Veröffentlichungsnummer: **0 088 979**

**A1**

(12) # EUROPÄISCHE PATENTANMELDUNG

(21) Anmeldenummer: 83102217.3

(22) Anmeldetag: 07.03.83

(51) Int. Cl.³: **C 02 F 1/44**
 **B 01 D 13/00**

(30) Priorität: 12.03.82 DE 3208926

(43) Veröffentlichungstag der Anmeldung:
 21.09.83 Patentblatt 83/38

(84) Benannte Vertragsstaaten:
 AT BE CH FR GB IT LI LU NL SE

(71) Anmelder: Herco W. Herrmann & Co. GmbH
 Planckstrasse 26
 D-7141 Freiberg (Neckar)(DE)

(72) Erfinder: Ardabili, Massud, Dipl.-Chem.
 Planckstrasse 26
 D-7141 Freiberg (Neckar)(DE)

(74) Vertreter: Becker, Maria, Dipl.-Phys.
 Auf dem Haigst 29
 D-7000 Stuttgart 70(DE)

(54) Vorrichtung zur Aufbereitung von wässrigen Lösungen, insbesondere Wasser.

(57) Die Erfindung betrifft eine Vorrichtung zur Aufbereitung von wässrigen Lösungen, insbesondere Wasser, mit Hilfe einer Umkehrosmosezelle (10). Es soll durch die Erfindung einer bakteriellen Kontamination entgegengewirkt werden. Dieses wird dadurch erreicht, dass parallel zum Eingangsventil (3) für das Rohwasser eine Umgehungsleitung (36) vorgesehen ist, durch die ein kontinuierlicher Durchfluss aufrechterhalten wird, welcher die Osmosezelle (10) ständig beaufschlagt.

EP 0 088 979 A1

0088979

Vorrichtung zur Aufbereitung von wässrigen Lösungen,
insbesondere Wasser.

Die Erfindung betrifft eine Vorrichtung zur Aufbereitung
von wässrigen Lösungen, insbesondere Wasser, unter
Verwendung einer Umkehrosmosezelle, die bei vorgegebenem
Druck von der aufzubereitenden Lösung durchflossen wird,
wobei die Rohflüssigkeitsleitung nur beim Betrieb der
Vorrichtung mittels eines Hauptventils für den Durchstrom
der Rohflüssigkeit freigegeben ist.

Beim Nichtbetrieb der Vorrichtung vermehren sich die in
der Rohflüssigkeit befindlichen Keime. Sie erzeugen
Toxine und können durch Algenbildung die Osmosezelle
blockieren. Um diesem Nachteil abzuhelfen, ist es bekannt,
auch bei Nichtbedarf von gereinigtem Wasser das Hauptventil zum Durchströmen der Anlage mittels einer Zeitschaltuhr kurzzeitig - z.B. stündlich - zu öffnen.

Diese Massnahme hat jedoch den Nachteil, dass nach bereits eingetretener Keimvermehrung durch Temperaturerhöhung am Modul die Keime des öfteren beim plötzlichen Wiedereinschalten der Anlage und vollem Druckanstieg zuerst durch das Umkehrosmosemodul gehen und die Reinwasserseite kontaminieren. Ferner verkürzen die ständigen Schaltmassnahmen die Lebensdauer der Module, Pumpen, Motoren usw.

Es ist auch bekannt, der bakteriellen Kontamination mittels einer in die Vorrichtung eingebauten UV-Anlage entgegenzuwirken. Eine derartige Anlage ist jedoch teuer und häufig nicht ausreichend wirksam.

Der Erfindung liegt die Aufgabe zugrunde, diese Nachteile zu vermeiden und eine einfache und wirtschaftliche Vorrichtung zu schaffen, mit der dem Übelstand der Kontamination in der stagnierenden Flüssigkeit wirksam entgegengetreten wird.

Diese Aufgabe wird bei einer Vorrichtung der eingangs beschriebenen Art erfindungsgemäss gelöst durch eine von der Rohflüssigkeitsleitung abgezweigte Umgehungsleitung, durch die kontinuierlich der Durchfluss einer geringen Teilstrommenge der Rohflüssigkeit aufrechterhalten und der Umkehrosmosezelle zugeführt wird.

Die Bemessung der Teilstrommenge liegt je nach Grösse der durchströmten Module zwischen ca. 2 % bei den grösseren und ca. 12 % bei den kleineren Typen.

Da durch diese Konstruktion überhaupt kein Stillstand der aufzubereitenden Flüssigkeit stattfindet, ist keinerlei Keimzahlvermehrung möglich.

Gemäss einer vorteilhaften Ausführungsform der Erfindung ist vorgesehen, dass die Umgehungsleitung nach dem Hauptventil in die Rohflüssigkeitsleitung einmündet. Dabei ist zweckmässig zur Einstellung der vorgegebenen Teilstrommenge in der Umgehungsleitung ein Ventil vorgesehen, das parallel zum Hauptventil angeordnet ist.

Derartige Vorrichtungen zur Aufbereitung von Flüssigkeiten, insbesondere zur Entsalzung und Reinigung von Wasser, werden beispielsweise in Klimaanlagen, als Raumbefeuchter, in der Datenverarbeitung oder in der Medizintechnik als Vorschaltgeräte von Dialysegeräten verwendet.

Die Erfindung ist in der nachfolgenden Beschreibung im Zusammenhang mit der anliegenden Zeichnung, in der ein Ausführungsbeispiel einer erfindungsgemässen Vorrichtung schematisch dargestellt ist, näher erläutert. Dabei werden die Ausführungen der Einfachheit halber auf Wasser bezogen. Es können aber auch andere wässrige Flüssigkeiten mit der erfindungsgemässen Vorrichtung aufbereitet werden.

Das Rohwasser tritt in die Leitung 20 ein und passiert zunächst einen Feinfilter 1, in welchem eine Feinstfiltration zur Beseitigung von Verunreinigungen erfolgt.

Um den Differenzdruck zur Messung des Durchgangswiderstandes zwecks Feststellung einer notwendigen Erneuerung der Filterkerze zu erkennen, sind an dessen Ein- und Ausgang Manometer 21, 22 vorgesehen.

- 4 -

0088979

In die Rohwasserleitung 20 ist nach dem Feinfilter 1 ein Ventil 3, vorzugsweise ein Magnetventil, geschaltet, welches bei Bedarf von gereinigtem Wasser, d.h. bei Inbetriebnahme der Vorrichtung, die Leitung 20 öffnet und das im Feinfilter 1 vorgereinigte Wasser dem Umkehrosmosemodul 10 zur weiteren Reinigung über einen Druckschalter 4 zuführt.

Der Druckschalter 4 schaltet bei Unterschreitung eines eingestellten Mindestdruckes in der Rohwasserzuleitung 20 lediglich das Motor-Pumpen-Aggregat 6, 7 ab. Darüber hinaus gibt es in der gesamten Anlage keine funktionsbedingten Abhängigkeiten oder Anpassungen. Die Pumpe 6 ist so beschaffen, dass auch bei Nichtbetrieb der Anlage, d.h. bei geschlossenem Ventil 3, ein Mindestdurchlauf bei abgeschaltetem Pumpenbetrieb möglich ist. Die Pumpe ist also bei Teillastbetrieb abgeschaltet und wird nur bei voller Last betrieben. Es ist dadurch ein wirtschaftlicher Betrieb der Anlage möglich.

Vor dem Modul 10 ist eine Druckregelung mittels eines insgesamt mit 24 gekennzeichneten Druckregelkreislaufs vorgesehen, der aus dem in der Leitung 20 befindlichen Motor-Pumpenaggregat 6, 7 sowie einem in einer Zweigleitung 26 angeschlossenen Manometer 9 und einem Druckregelventil 8 besteht. Hierdurch wird für das Umkehrosmosemodul 10 zur Aufbereitung des Wassers ein vorgegebener Druck aufgebaut. Die Einstellung des Ventils 8 erfolgt bei der ersten Inbetriebsetzung.

Das Permeat verlässt das Modul 10 über eine Leitung 27 und wird über die Leitung 28, in welcher sich ein Strömungsmesser 11 und eine Messzelle 12 mit dem Ausgang 14 befinden, zum Verbrauch weitergeleitet. Der Reinheitsgrad
des Permeates wird durch den Leitwertmesser 13 angezeigt.

Zur Vermeidung eines auf die Permeatseite des Moduls
wirkenden Druckstosses, wie er regelmässig bei plötzlichen
Absperrungen des Permeatstromes auftritt, ist über eine
Leitung 29 und ein federbelastetes Sicherheitsventil 37
eine Verbindung zur Rohwasserleitung 20 geschaffen.
Hierdurch werden sonst mögliche Schädigungen der Module 10
vermieden.

Das Konzentrat wird über eine Leitung 30, in der sich
ein Manometer 9, ein Regelventil 15 und ein Strömungsmesser 11 befinden, zum Kanal 16 abgeleitet oder je nach
Sachlage und Beschaffenheit des Konzentrates einer
Wiederverwendung zugeführt.

Die Einstellung des Verhältnisses Permeat : Konzentrat
wird einmalig bei der ersten Inbetriebsetzung über das
Ventil 15 vorgenommen und richtet sich nach dem gewünschten
Reinheitsgrad des Permeats.

Wie aus dem dargestellten Fließschema ersichtlich, ist
eingangsseitig eine Umgehungsleitung 36 zur Rohwasserleitung 20 vorgesehen. Mittels eines Ventils 2 ist eine
durch diese Zweigleitung strömende Teilstrommenge
einzustellen. Je nach der Grösse der Umkehrosmosezelle
ist der Teillaststrom 2 % bis 12 % des durch die Leitung 20
fliessenden Vollaststroms. Die Bypassleitung 36 mündet
in die Rohwasserleitung 20 unter Umgehung des Ventils 3
ein, so dass eine ständige geringe Strömung vorhanden ist,
durch welche das Umkehrosmosemodul 10 ununterbrochen durchströmt wird. Das Permeat ist absolut keimfrei,
da eine Stagnation des Wassers zu keiner Zeit stattgefunden hat.

Vorrichtung zur Aufbereitung von wässrigen Lösungen,
insbesondere Wasser.

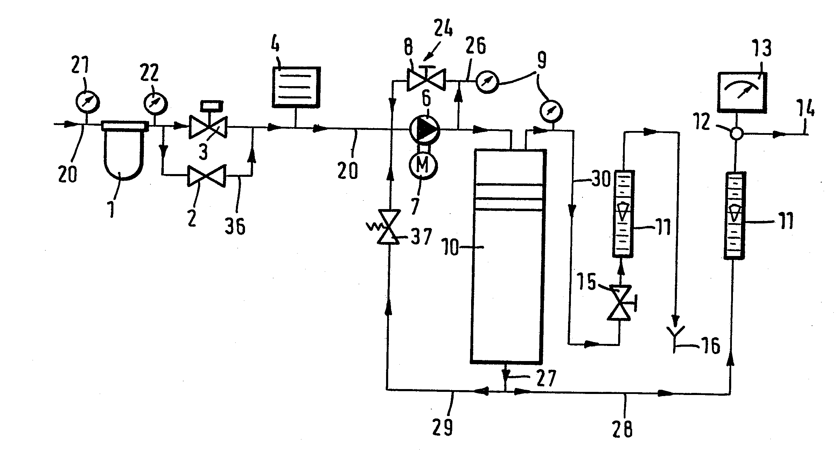

Patentansprüche:

1. Vorrichtung zur Aufbereitung von wässrigen Lösungen,
insbesondere Wasser, unter Verwendung einer Umkehrosmosezelle, die bei vorgegebenem Druck von der aufzubereitenden Lösung durchflossen wird, wobei die
Rohflüssigkeitsleitung nur beim Betrieb der Vorrichtung
mittels eines Hauptventils für den Durchstrom der
Rohflüssigkeit freigegeben ist,
g e k e n n z e i c h n e t   d u r c h
eine von der Rohflüssigkeitsleitung (20) abgezweigte
Umgehungsleitung (36), durch die kontinuierlich der
Durchfluss einer geringen Teilstrommenge der Rohflüssigkeit aufrechterhalten und der Umkehrosmosezelle (10)
zugeführt wird.

2. Vorrichtung nach Anspruch 1, dadurch gekennzeichnet, dass die Umgehungsleitung (36) nach dem Hauptventil (3) in die Rohflüssigkeitsleitung (20) einmündet.

3. Vorrichtung nach den Ansprüchen 1 und 2, dadurch gekennzeichnet, dass zur Einstellung der vorgegebenen Teilstrommenge in der Umgehungsleitung (36) ein Ventil (2) vorgesehen ist, das parallel zum Hauptventil (3) angeordnet ist.

4. Vorrichtung nach einem der vorhergehenden Ansprüche, dadurch gekennzeichnet, dass die Teilstrommenge in der Umgehungsleitung (36) ca. 2 % bis ca. 12 % der Gesamtstrommenge beträgt.

0088979

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| X | US-A-3 856 676 (RAYPAK INC.) * Figuren 5,6; Anspruch 3; Spalte 3, Zeile 57 - Spalte 4, Zeile 11; Spalte 6, Zeile 33 - Spalte 7, Zeile 3; Spalte 4, Zeilen 30-37 * | 1,2 | C 02 F 1/44 B 01 D 13/00 |
| | --- | | |
| A | DE-A-2 627 366 (E. ALHÄUSER) * Figur 1; Ansprüche 1,11; Seite 10, Zeilen 8-31; Seite 14, Zeilen 1-14 * | 1 | |
| | --- | | |
| A | FR-A-2 401 877 (BLUTSPENDEDIENST DER LANDESVERBANDE DES DEUTSCHEN ROTEN KREUZES NIEDERSACHSEN OLDENBURG UND BREMEN GmbH) * Figur 3; Seite 5, Zeile 5 - Seite 6, Zeile 4 * | 1 | |
| | | | **RECHERCHIERTE SACHGEBIETE (Int. Cl. ³)** |
| | --- | | |
| P | DE-A-3 106 772 (CILLICHEMIE E. VOGELMANN GmbH & CO.) * Zusammenfassung; Figur 1; Anspruch 13; Seite 16, Zeilen 3-20 * | 1 | B 01 D C 02 F |
| | ----- | | |

Der vorliegende Recherchenbericht wurde für alle Patentansprüche erstellt.

| Recherchenort DEN HAAG | Abschlußdatum der Recherche 22-07-1983 | Prüfer HOORNAERT P.G.R.J. |
|---|---|---|